# EUROPÄISCHE PATENTANMELDUNG

(11) **EP 1 006 175 A1**
(43) Veröffentlichungstag der Anmeldung: **07.06.2000**
(21) Anmeldenummer: 99123403.0
(22) Anmeldetag: 24.11.1999
(51) Int. Cl.: C11C 3/12, C07C 57/12, C07C 51/36, C07C 51/43, C07C 51/44, A61K 7/00, A61K 31/23, C11D 1/04, C07C 69/58, C07C 33/025

(54) **Oxidationsstabile Oleine**

(30) Priorität: 03.12.1998 DE 19855765
(71) Anmelder: Cognis Deutschland GmbH, 40589 Düsseldorf (DE)
(72) Erfinder: Strube, Albert, Dr., 41470 Neuss (DE); Petersen, Heinrich, 40591 Düsseldorf (DE); Christoph, Ralf, Dr., 40764 Langenfeld (DE); Steinberner, Udo, Dr., 40724 Hilden (DE)

(57) **Zusammenfassung**

Vorgeschlagen werden oxidationsstabile Oleine mit einer Iodzahl von 60 bis 130, einem Trübungspunkt unterhalb von 11°C, die im wesentlich ungesättigte Fettsäuren enthalten, dabei mehr als 65 Gew.-% Ölsäure, jedoch weniger als 16 Gew.-% Linolsäure aufweisen, dadurch erhältlich, daß man Raffinationsfettsäuren
(a) partiell härtet,
(b) die resultierenden Härtungsprodukte einer Trennung in im wesentlichen ungesättigte Fettsäuren (Oleine) und im wesentlichen gesättigte Fettsäuren unterwirft und
(c) anschließend das erhaltene Roholein destillativ aufarbeitet.

## Beschreibung

### Gebiet der Erfindung

Die vorliegende Erfindung betrifft oxidationsstabile ungesättigte Fettsäuren ( 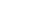 Oleine") pflanzlichen Ursprungs, ein Verfahren zu ihrer Herstellung sowie die Verwendung der Oleine als Ausgangsstoffe zur Herstellung oberflächenaktiver Substanzen.

### Stand der Technik

Oleine sind technische Gemische von ungesättigten und gesättigten Fettsäuren, die überwiegend Ölsäure enthalten. Üblicherweise werden Oleine aus den natürlich vorkommenden Fetten und Ölen, insbesondere tierischen Ölen gewonnen. Für die Gewinnung von Olein aus Talg ist aus dem Stand der Technik beispielsweise folgendes Verfahren bekannt: Die Triglyceride werden üblicherweise einer Druckspaltung unterworfen und anschließend die resultierenden Gemische der verschiedenen Fettsäuren beispielsweise mittels einer Umnetztrennung in einen überwiegend gesättigten (Stearin) und einen überwiegend ungesättigten Anteil (Olein) getrennt. Das erhaltene Olein kann direkt oder nach weiterer Aufreinigung durch Destillation oder nach entsprechender Derivatisierung beispielsweise in Schmiermitteln, Kosmetika, Salben, Nahrungsmitteln und/ oder Futtermitteln eingesetzt werden. Üblicherweise erfolgt die Herstellung von Olein ausgehend von Talg. Bei Durchführung dieses Verfahrens mit Ausgangsstoffen die einen erhöhten Anteil an mehrfach ungesättigten Fettsäuren enthalten, wie dies insbesondere bei pflanzlichen Ausgangsstoffen der Fall ist, erhält man Oleine mit deutlich schlechteren Eigenschaften insbesondere bezüglich der Oxidationsstabilität. Bislang ist pflanzliches Olein beispielsweise aus Palmöl oder Olivenöl zugänglich. Oleine auf Basis von Olivenöl weisen im allgemeinen unerwünscht hohe Trübungspunkte oberhalb 15 und sogar oberhalb von 20 °C auf. Des weiteren wirkt sich der hohe Anteil an mehrfach ungesättigten Fettsäuren negativ auf die Oxidationsstabilität aus. Die Gewinnung von Olein aus Palmkernöl ist durch die komplexe Aufarbeitung sehr aufwendig und teuer außerdem sind nur begrenzte Mengen auf diesem Wege erhältlich. Die in den letzten Jahren zunehmende Diskussion über BSE im Zusammenhang mit Produkten tierischen Ursprungs führte insbesondere im Bereich der Kosmetik dazu, nach alternativen Produkten, vorzugsweise auf pflanzlicher Basis zu suchen. Der einfache Ersatz von Talg beispielsweise durch Palmöl ist aufgrund der bereits erwähnten Unterschiede bezüglich des Fettsäuregehalts und -verteilung mit Qualitätsproblemen der erhaltenen Oleine verbunden.

Aufgabe der vorliegenden Erfindung war es daher ein technisch leicht durchzuführendes Verfahren zur Herstellung verbesserter Oleine zu entwickeln, durch das ungesättigte Fettsäuren auf Basis pflanzlicher Rohstoffe auch in großen Mengen zugänglich ist. Die verbesserten Oleine sollten sich insbesondere durch einen Trübungspunkt unterhalb von 11°C, eine hohe Oxidationsstabilität sowie eine gute Farbstabilität auszeichnen.

### Beschreibung der Erfindung

Gegenstand der Erfindung sind oxidationsstabile Oleine mit einer Iodzahl von 60 bis 130, einem Trübungspunkt unterhalb von 11°C, die im wesentlich ungesättigte Fettsäuren enthalten, und dabei mehr als 65 Gew.-% Ölsäure, jedoch weniger als 16 Gew.-% Linolsäure aufweisen, die man dadurch erhält, daß man Raffinationsfettsäuren
(a) partiell härtet,
(b) die resultierenden Härtungsprodukte einer Trennung in im wesentlichen ungesättigte Fettsäuren (Oleine) und im wesentlichen gesättigte Fettsäuren unterwirft und
(c) anschließend das erhaltene Roholein destillativ aufarbeitet.

Überraschenderweise wurde festgestellt, daß sich durch die Kombination von partieller Härtung und Olein/Stearintrennung nach destillativer Aufarbeitung Oleine herstellen lassen, die sich durch niedrige Trübungspunkte auszeichnen, insbesondere ausgehend von pflanzlichen Rohstoffen, die große Mengen an mehrfach ungesättigten Fettsäuren enthalten. Die üblicherweise bei der partiellen Härtung entstehenden trans-Fettsäuren werden gemäß dem Verfahren der vorliegenden Erfindung im Rahmen der Olein-, Stearintrennung, vorzugsweise mittels Netzmitteltrennung gleichzeitig mit den gesättigten Anteilen abgetrennt. Insbesondere überraschend war, daß die erfindungsgemäß hergestellten Oleine deutlich oxidationsstabiler waren als die aus dem Stand der Technik bekannten Produkte ― insbesondere tierischen Ursprungs - obwohl der Linolsäuregehalt bei den erfindungsgemäßen Oleinen höher liegt.

Ein weiterer Gegenstand der Erfindung betrifft ein Verfahren zur Herstellung von oxidationsstabilen Oleinen mit einer Iodzahl von 60 bis 130, einem Trübungspunkt unterhalb von 11°C, die im wesentlich ungesättigte Fettsäuren enthalten, und dabei mehr als 65 Gew.-% Ölsäure, jedoch weniger als 16 Gew.-% Linolsäure aufweisen, welches sich dadurch auszeichnet, daß man Raffinationsfettsäuren
(a) partiell härtet,
(b) die resultierenden Härtungsprodukte einer Trennung in im wesentlichen ungesättigte Fettsäuren (Oleine) und im wesentlichen gesättigte Fettsäuren unterwirft und
(c) anschließend das erhaltene Roholein destillativ aufarbeitet.

### Olein

Im Rahmen der vorliegenden Erfindung sind unter Oleinen Mischungen von überwiegend ungesättigten Fettsäuren mit Anteilen gesättigter Fettsäuren zu verstehen. Zur Definition von Oleinen siehe auch **Römpp 9. Aufl., Bd.4, 3106.** Die erfindungsgemäßen Oleine sind pflanzlicher Herkunft, vorzugsweise auf Basis von neuem Sonnenblumenöl (das auch als Sonnenblumenöl der neuen Züchtung bezeichnet wird) und weisen einen Ölsäuregehalt von mehr als 65, vorzugsweise mehr als 75 und insbesondere mehr als 83 Gew.-% auf. Sie zeichnen sich durch ihre hohe Farb-und Oxidationsstabilität sowie einen geringen Eigengeruch aus. Vorzugsweise weisen die erfindungsgemäßen Oleine eine Oxidationsstabilität von mehr als 45, insbesondere mehr als 55 und besonders bevorzugt mehr als 65 Stunden, bestimmt mittels einem Oxidographen in Anlehnung an den Sylvester-Test N.D. von 1941, auf. Diese Eigenschaften lassen sich nur teilweise auf den relativ geringen Gehalt an mehrfach ungesättigten Verbindungen zurückführen, insbesondere einen Linolsäuregehalt von weniger als 16, vorzugsweise unterhalb von 15 und insbesondere kleiner 7 Gew.-%. Gleichzeitig liegt die Iodzahl der erfindungsgemäßen Oleine zwischen 60 und 130 und vorzugsweise zwischen 75 bis 115. Der Trübungspunkt der erfindungsgemäßen Oleine liegt unter 11, vorzugsweise unter 8 und insbesondere unter 5 °C. Weiterhin enthalten die erfindungsgemäßen Oleine nur geringe Anteile gesättigter Fettsäuren, so liegt der Gehalt an Palmitinsäure unter 5, insbesondere unterhalb von 4 und vorzugsweise unterhalb von 3 Gew.-% während der Stearinsäuregehalt weniger als 4, vorzugsweise weniger als 2 und insbesondere weniger als 1 Gew.-% beträgt.

### Raffinationsfettsäuren

Als Raffinationsfettsäuren kommen erfindungsgemäß alle Fettsäuremischungen in Frage, die durch enzymatische Hydrolyse ( 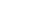 Ranzigwerden") von natürlichen Triglyceriden, insbesondere pflanzlichen Fetten und Ölen gebildet werden und dann bei der Raffination dieser Stoffe anfallen. Unter Raffinationsfettsäuren sind dabei vorzugsweise aliphatische Carbonsäuren der Formel **(I)** zu verstehen, 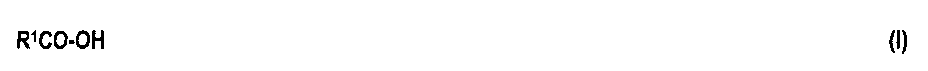 in der R¹CO für einen aliphatischen, linearen oder verzweigten Acylrest mit 6 bis 22 Kohlenstoffatomen und 0 und/oder 1, 2 oder 3 Doppelbindungen steht. Insbesondere handelt es sich um lineare Carbonsäuren pflanzlichen Ursprungs. Typische Beispiele sind Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Elaeostearinsäure, Arachinsäure, Gadoleinsäure, Behensäure und Erucasäure. Vorzugsweise werden die Raffinationsfettsäuren bei der Entsäuerung von pflanzlichen Ölen, wie z.B. Sonnenblumenöl, Canolaöl oder Sojaöl gewonnen. Besonders bevorzugt werden solche Raffinationsfettsäuren eingesetzt, welche durch Destillation und/oder Fraktionierung von unverseifbaren Anteilen befreit worden sind.

### Partielle Härtung

Wesentlich für die vorliegende Erfindung ist die Durchführung einer partiellen Härtung nach der Abtrennung der Raffinationsfettsäuren von den Fellen und Ölen. Unter partieller Härtung im Rahmen der vorliegenden Erfindung ist die Härtung der höher ungesättigten Anteile, insbesondere Linolsäure in einfach ungesättigte Ölsäure zu verstehen. Gleichzeitig erfolgt die Härtung eines gewissen Anteils der Ölsäure zu Stearinsäure, wobei jedoch der Gesamtölsäuregehalt in etwa konstant bleibt. Durch Überführung der höher ungesättigten Anteile, insbesondere der Linolsäure in die einfach ungesättigte Ölsäure ist es nun möglich ausgehend von natürlichen, insbesondere pflanzlichen Rohstoffen Oleine mit einer erhöhten Oxidations- und Farbstabilität zu erhalten. Gleichzeitig wird durch die Erhöhung des Stearinanteils eine schärfere Trennung in Olein-und Stearinanteil beispielsweise im Rahmen des Umnetzverfahrens ermöglicht. Überraschenderweise hat sich jedoch gezeigt, daß der geringe Linolsäuregehalt nicht allein die hohe Oxidationsstabilität bedingt, da auf anderen Wegen erhaltene Oleine, beispielsweise tierischen Ursprungs, mit geringeren Linolsäuregehalten oxidationsempfindlicher sind. Erfindungsgemäß wird die partielle Härtung mit einem üblichen aus dem Stand der Technik für solche Verfahren bekannten Katalysator durchgeführt. Bevorzugt handelt es sich um einen Katalysator auf Basis von Nickel. Die partielle Härtung wird bei einer Temperatur von vorzugsweise 120 bis 180 insbesondere 130 bis 150 °C und einem Wasserstoffdruck von 1 bis 4, insbesondere 2 bis 3 bar durchgeführt. Bevorzugt weisen die Fette und Öle bzw. die Spaltfettsäuren nach der partiellen Härtung Iodzahlen im Bereich von 60 bis 130, insbesondere 75 bis 115 auf.

### Olein-/ Stearintrennung

Zur Trennung der ungesättigten und gesättigten Fettsäuren sind aus dem Stand der Technik verschiedene Verfahren bekannt, die im Rahmen des erfindungsgemäßen Verfahrens angewendet werden können. Die Trennung kann beispielsweise anhand des sogenannten 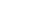 Alfa-Laval"-Verfahrens erfolgen, wie es in **J.Am.Oil.Chem.Soc., 61, 219 ff (1984)** beschrieben ist, oder mittels Lösungsmitteltrennung, dem sogenannten Emersol-Verfahren, durchgeführt werden. Besonders bevorzugt erfolgt die Trennung in Olein und Stearin-Anteil mittels Umnetzverfahren, welches am Beispiel der Talgfettsäure gut untersucht ist. Bei diesem Verfahren wird das Fettsäuregemisch zunächst abgekühlt, bis die Stearinsäure in der flüssigen Ölsäure auskristallisiert und anschließend ein Netzmittel, beispielsweise eine wäßrige Alkylsulfatlösung hinzugegeben, um die Ölsäure zu emulgieren. Durch folgende Zentrifugation werden Ölsäurephase und Stearin-/Wasserphase getrennt (**Fat Sci.Technol. 89, 237 (1987)**).

### Destillation

Zur Aufreinigung des Roholeins schließt sich vorzugsweise eine Destillation bei einer maximalen Sumpftemperatur von 260 °C und einem Vakuum von 2 bis 10 mbar, insbesondere 3 mbar an. Die erfindungsgemäßen Oleine lassen sich zur Herstellung von Derivaten, insbesondere Oleinestern, - amiden und/oder ungesättigten Alkoholen verwenden, welche über die gleichen hervorragenden Eigenschaften im Bezug auf Farb-, Geruchs- und Oxidationsstabilität verfügen wie die Oleine selber. Weiterhin lassen sich sowohl die Oleine selber als auch deren Derivate gemäß der vorliegenden Erfindung in Wasch- und Reinigungsmitteln sowie kosmetischen und/oder pharmazeutischen Formulierungen einsetzen. Eine bevorzugte Verwendung der Oleine ist deren Hydrierung zur Herstellung von ungesättigten Alkoholen. Insbesondere bevorzugte Ester die aus den erfindungsgemäßen Oleinen hergestellt werden können, sind beispielsweise Sorbitanoleat, Cetyloleat, Oleyloleat, und/oder Decyloleat.

### Beispiele

**Beispiel 1.** Eine Raffinationsfettsäure auf Basis von Sonnenblumenöl neuer Züchtung wurde über einem handelsüblichen Nickelkatalysator bei einem Druck von 1 bar, bei 135°C für 30 Minuten angehärtet. Anschließend wurde die Fettsäuremischung per Umnetztrennung gemäß der Bedingungen angegeben in Fat. Sci.Technol. 89, 237 (1987) in einen Olein- und einen Stearinanteil getrennt und bei einer Sumpftemperatur von 260°C und 3 mbar das Roholein destilliert. Die Produkteigenschaften sind Tabelle 1 zu entnehmen
**Vergleichsbeispiel V1.** Eine Raffinationsfettsäure auf Basis Sonnenblumenöl wurde per Umnetztrennung analog Beispiel 1 in Olein- und Stearinanteil getrennt und das Roholein bei einer Sumpftemperatur von 260 °C und einem Druck von 3 mbar destilliert. Die Produkteigenschaften sind Tabelle 1 zu entnehmen
**Vergleichsbeispiel V2.** Neues Sonnenblumenöl wurde gemäß den zuvor beschriebenen Beispielen einer Fettspaltung und Umnetztrennung unterworfen und anschließend destilliert, jedoch nicht angehärtet. Die Produkteigenschaften sind Tabelle 1 zu entnehmen
**Vergleichsbeispiel V3.** Palmöl wurde ― ohne Härtung ― einer Fettspaltung, Umnetztrennung und Destillation unterworfen. Die Produkteigenschaften sind Tabelle 1 zu entnehmen

**Tabelle 1**

| Versuchsergebnisse | | | | |
|---|---|---|---|---|
| | **1** | **V1** | **V2** | **V3** |
| ***C-Kettenverteilung [GC-Flächen-%]*** | | | | |
| < C₁₄ | - | - | - | 0,8 |
| C₁₄ | - | - | - | 1,1 |
| C₁₅ | - | - | - | 0,1 |
| C₁₆ | 2,7 | 3,9 | 3,4 | 4,7 |
| C₁₆, einfach ungesättigt | 0,2 | 0,2 | 0,1 | 0,4 |
| C₁₇ | - | - | - | 0,1 |
| C₁₈ | 1,2 | 1,3 | 2,0 | 1,1 |
| C₁₈, einfach ungesättigt | 84,1 | 65,6 | 89,1 | 71,9 |
| C₁₈, zweifach ungesättigt | 9,8 | 19,4 | 5,2 | 19,7 |
| C₁₈, dreifach ungesättigt | 0,2 | 7,8 | - | 0,4 |
| C₂₀ | 0,2 | 0,3 | 0,2 | 0,2 |
| C₂₀, einfach ungesättigt | 1,3 | 1,3 | 0,2 | 0,3 |
| C₂₂ | 0,1 | 0,1 | 0,1 | - |
| C₂₂, einfach ungesättigt | 0,2 | 0,1 | - | - |
| % trans berechnet auf Ölsäure | 15,5 | 4,0 | 0,9 | 0,5 |
| ***Säurezahl*** | 198 | 198 | 200 | 201 |
| ***Iodzahl*** | 96 | 116 | 89 | 101 |
| ***Trübungspunkt [°C]*** | 7,5 | 3,5 | 10,5 | 6,0 |
| ***Oxidationsstabilität [h]*** | 61 | 40 | 45 | 43 |

| ***Lovibond-Farbzahl (5 ¼"-Küvette)*** | | | | |
|---|---|---|---|---|
| - rot | 2,1 | 4,1 | 1,5 | 4,6 |
| - gelb | 0,1 | 0,3 | 0,1 | 0,3 |

## Patentansprüche

1. Oxidationsstabile Oleine mit einer Iodzahl von 60 bis 130, einem Trübungspunkt unterhalb von 11°C, die im wesentlich ungesättigte Fettsäuren enthalten, dabei mehr als 65 Gew.-% Ölsäure, jedoch weniger als 16 Gew.-% Linolsäure aufweisen, dadurch erhältlich, daß man Raffinationsfettsäuren
(a) partiell härtet,
(b) die resultierenden Härtungsprodukte einer Trennung in im wesentlichen ungesättigte Fettsäuren (Oleine) und im wesentlichen gesättigte Fettsäuren unterwirft und
(c) anschließend das erhaltene Roholein destillativ aufarbeitet.

2. Oleine nach Anspruch 1, **dadurch gekennzeichnet,** daß sie im Oxidographentest gemäß Sylve-ster-Test N.D. von 1941 eine Oxidationsstabilität von mehr als 45 Stunden aufweisen.

3. Verfahren zur Herstellung von oxidationsstabilen Oleinen mit einer Iodzahl von 60 bis 130, einem Trübungspunkt unterhalb von 11°C, die im wesentlich ungesättigte Fettsäuren enthalten, dabei mehr als 65 Gew.-% Ölsäure jedoch weniger als 16 Gew.-% Linolsäure aufweisen, bei dem man Raffinationsfettsäuren
(a) partiell härtet,
(b) die resultierenden Härtungsprodukte einer Trennung in im wesentlichen ungesättigte Fettsäuren (Oleine) und im wesentlichen gesättigte Fettsäuren unterwirft und
(c) anschließend das erhaltene Roholein destillativ aufarbeitet.

4. Verfahren nach Anspruch 3, **dadurch gekennzeichnet,** daß man Raffinationsfettsäuren auf Basis von Sonnenblumenöl, Canolaöl oder Sojaöl einsetzt.

5. Verfahren nach den Ansprüchen 3 und/oder 4, **dadurch gekennzeichnet,** daß man Raffinationsfettsäuren einsetzt, welche man durch Destillation und/oder Fraktionierung von unverseifbaren Anteilen befreit hat.

6. Verwendung der Oleine gemäß Anspruch 1 in Wasch- und Reinigungsmitteln sowie kosmetischen und/ oder pharmazeutischen Formulierungen.

7. Verwendung der Oleine gemäß Anspruch 1 als Ausgangsstoffe zur Herstellung von ungesättigten Alkoholen ( 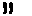 Ocenolen") oder ungesättigten Estern.
